(19) **Europäisches Patentamt / European Patent Office / Office européen des brevets**

(11) **EP 2 477 552 B1**

(12) # FASCICULE DE BREVET EUROPEEN

(45) Date de publication et mention de la délivrance du brevet:
**20.08.2014 Bulletin 2014/34**

(51) Int Cl.:
**G10L 15/24** (2013.01)   **A61B 8/08** (2006.01)
**G10L 21/10** (2013.01)   **A61B 5/00** (2006.01)

(21) Numéro de dépôt: **10760594.1**

(22) Date de dépôt: **15.09.2010**

(86) Numéro de dépôt international:
**PCT/EP2010/005651**

(87) Numéro de publication internationale:
**WO 2011/032688 (24.03.2011 Gazette 2011/12)**

(54) **DISPOSITIFS DE RECONSTITUTION DE LA PAROLE PAR SONDAGE ULTRASONORE DE L'APPAREIL PHONATOIRE**

VORRICHTUNGEN ZUR SPRACHREKONSTRUKTION MITTELS ULTRASCHALLSONDIERUNG DES STIMMAPPARATS

DEVICES FOR RECONSTRUCTING SPEECH BY ULTRASONICALLY PROBING THE VOCAL APPARATUS

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **17.09.2009 FR 0904444**

(43) Date de publication de la demande:
**25.07.2012 Bulletin 2012/30**

(73) Titulaires:
• **Université Pierre et Marie Curie (Paris 6)**
**75005 Paris (FR)**
• **Centre National de la Recherche Scientifique**
**75016 Paris (FR)**

(72) Inventeurs:
• **HUEBER, Thomas**
**F-75015 Paris (FR)**
• **DENBY, Bruce**
**F-75013 Paris (FR)**
• **DREYFUS, Gérald**
**F-91190 Gif sur Yvette (FR)**
• **DUBOIS, Rémi**
**F-75015 Paris (FR)**
• **ROUSSEL, Pierre**
**F-75005 Paris (FR)**

(74) Mandataire: **Parzy, Benjamin Alain et al**
**Cabinet Boettcher**
**16, rue Médéric**
**75017 Paris (FR)**

(56) Documents cités:
**US-A1- 2002 024 675**

• **SCOBBIE, J.M. ET AL: "Head-probe stabilisation in ultrasound tongue imaging using a headset to permit natural head movement", PROCEEDINGS OF THE EIGHTH INTERNATIONAL SEMINAR ON SPEECH PRODUCTION (ISSP), 10 décembre 2008 (2008-12-10), pages 373-376, XP002566548, Strasbourg**
• **STONE M.: "A Guide to Analyzing Tongue Motion from Ultrasound Images", CLINICAL LINGUISTICS AND PHONETICS, vol. 19, no. 6-7, 1 septembre 2005 (2005-09-01), pages 455-502, XP002566577,**
• **STONE M ET AL: "A HEAD AND TRANSDUCER SUPPORT SYSTEM FOR MAKING ULTRASOUND IMAGES OF TONGUE/JAW MOVEMENT", THE JOURNAL OF THE ACOUSTICAL SOCIETY OF AMERICA, AMERICAN INSTITUTE OF PHYSICS FOR THE ACOUSTICAL SOCIETY OF AMERICA, NEW YORK, NY, US, vol. 98, no. 6, 1 décembre 1995 (1995-12-01), pages 3107-3112, XP000545621, ISSN: 0001-4966 cité dans la demande**

• DENBY B ET AL: "Speech synthesis from real time ultrasound images of the tongue", ACOUSTICS, SPEECH, AND SIGNAL PROCESSING, 2004. PROCEEDINGS. (ICASSP ' 04). IEEE INTERNATIONAL CONFERENCE ON MONTREAL, QUEBEC, CANADA 17-21 MAY 2004, PISCATAWAY, NJ, USA,IEEE, PISCATAWAY, NJ, USA, vol. 1, 17 mai 2004 (2004-05-17), pages 685-688, XP010717721, ISBN: 978-0-7803-8484-2 cité dans la demande

## Description

L'invention est relative à un dispositif de reconnaissance et/ou de reconstitution de la parole par sondage ultrasonore de l'appareil phonatoire.

ARRIERE-PLAN DE L'INVENTION

Il a été proposé de faire de la reconnaissance ou de la reconstitution de la parole par imagerie ultrasonore de l'appareil phonatoire. On consultera par exemple l'article intitulé « SPEECH SYNTHESIS FROM REAL TIME ULTRASOUND IMAGES OF THE TONGUE » par Bruce Denby et Maureen Stone, publié à l'occasion de la conférence 2004 IEEE International Conference on Acoustics, Speech, and Signal Processing -ICASSP04- Montreal, May 17-21 2004. A cet effet, on utilise en général une sonde ultrasonore mettant en oeuvre une série d'émetteurs/récepteurs ultrasonores, en pratique des transducteurs de type piézoélectrique qui sont adaptés à émettre des ondes ultrasonores et recevoir des ondes réfléchies pour les transformer en signal électrique, ou, si l'application le permet, un unique transducteur ultrasonore.

Il a été également proposé l'utilisation d'ultrasons à basse fréquence se propageant dans l'air pour une application similaire. Le ou les transducteurs ultrasonores sont avantageusement associés à un téléphone portable de sorte que ceux-ci se trouvent à proximité de l'appareil phonatoire lorsque l'utilisateur téléphone.

Cependant, la position relative du ou des capteurs ultrasonores par rapport à la tête de l'utilisateur ne peut être connue avec précision et dépend essentiellement de la façon dont l'utilisateur tient le dispositif. L'analyse des signaux des capteurs ultrasonores en est rendue plus difficile.

Pour éviter ce problème, il a été proposé dans certains dispositifs expérimentaux d'immobiliser la tête de l'utilisateur par rapport aux capteurs ultrasonores, de sorte que l'analyse des signaux générés par les capteurs ultrasonores ne soit affectée d'aucune incertitude quant à la position de ces derniers par rapport à la tête (voir notamment le dispositif HATS, pour « Head and Transducer Support System », visible à l'adresse suivante : http://speech.umaryland.edu/ahats.html, et décrit dans Stone, M., and Davis, E. (1995) "A Head and Transducer Support System for Making Ultrasound Images of Tongue/Jaw Movement," Journal of the Acoustical Society of America, 1995 98 (6), pp. 3107-3112.

Dans le dispositif proposé, une sonde ultrasonore est positionnée sous la mâchoire inférieure, et ne bouge pas avec cette dernière. Sa position par rapport à un repère donné est donc déterminée avec certitude, la tête étant elle-même immobilisée et sa position déterminée dans ledit repère, de sorte que l'on sait à tout moment sous quel angle d'incidence les ondes ultrasonores sont envoyées vers l'appareil phonatoire, ce qui facilite l'analyse des signaux. Cependant, ce type de dispositif n'est bien sûr pas utilisable en pratique dans la vie quotidienne.

Un dispositif similaire est proposé dans l'article « Head-Probe Stabilisation in Ultrasound Tongue Imaging Using a Headset to Permit Natural Head Movement » par Scobbie, J. M. ET AL, Proceedings of the Eight International Seminar on Speech Production (ISSP), 10 décembre 2008.

OBJET DE L'INVENTION

L'invention vise à proposer un dispositif portable de reconnaissance et/ou de reconstruction de la parole par sondage ultrasonore de l'appareil phonatoire, dans lequel l'analyse des signaux du ou des capteurs ultrasonores est facilitée.

BREVE DESCRIPTION DE L'INVENTION

En vue de la réalisation de ce but, on propose un dispositif portable de reconnaissance et/ou de reconstruction de la parole par sondage ultrasonore de l'appareil phonatoire, le dispositif comportant au moins un transducteur ultrasonore pour générer une onde ultrasonore et recevoir une onde réfléchie par l'appareil phonatoire, et des moyens d'analyse d'un signal généré par le transducteur ultrasonore. Selon l'invention, le dispositif comporte des moyens de localisation pour déterminer une position relative du transducteur ultrasonore par rapport au crâne de l'utilisateur.

Le transducteur ultrasonore n'étant pas fixe par rapport à l'appareil phonatoire, il importe de déterminer quel est son mouvement dans l'espace par rapport à un repère lié au crâne de l'utilisateur, afin de déterminer l'angle avec lequel les ondes ultrasonores viennent frapper l'appareil phonatoire, en particulier les éléments articulateurs de celui-ci. La connaissance de l'angle d'incidence permet de dissocier le mouvement du transducteur ultrasonore de celui des éléments articulateurs, qui est seul utile pour la reconnaissance ou la reconstruction de la parole. L'analyse du signal en est grandement facilitée.

BREVE DESCRIPTION DES FIGURES

L'invention sera mieux comprise à la lumière de la description qui suit des modes particuliers de réalisation de l'invention au moyen des figures des dessins annexés, parmi lesquelles :

- la figure 1 est une vue en perspective représentant un dispositif selon un premier mode particulier de réalisation de l'invention, porté par un utilisateur ;
- la figure 2 est une vue en perspective représentant un dispositif selon un deuxième mode particulier de réalisation de l'invention, porté par un utilisateur ;
- La figure 3 est une vue schématique en perspective montrant comment on peut déduire de la mesure de trois composantes de l'accélération de la pesanteur une orientation d'un trièdre lié à l'accéléromètre.

## DESCRIPTION DETAILLEE DE L'INVENTION

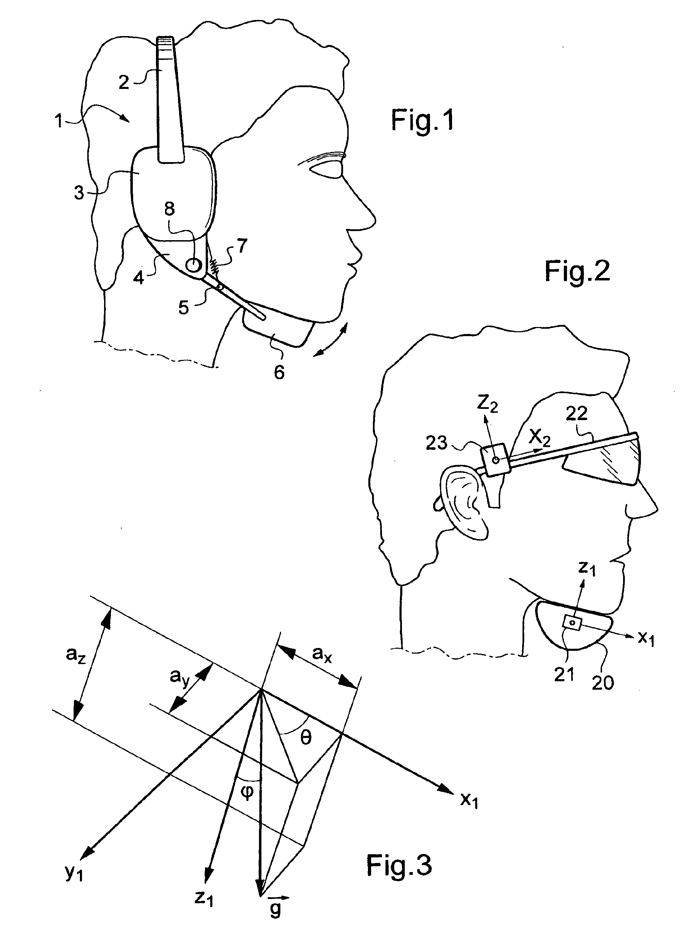

En référence à la figure 1, le dispositif de l'invention comporte un casque 1 comportant un arceau 2 portant des écouteurs 3. L'un des écouteurs comporte une extension inférieure 4 sur laquelle un bras 5 est monté pivotant selon un axe d'articulation sensiblement parallèlement à l'axe d'articulation de la mâchoire mobile sur le crâne. L'extrémité du bras 5 porte un capteur ultrasonore 6 qui vient se placer sous la mâchoire inférieure et qui est rappelé vers celle-ci par un ressort 7 attelé entre le bras 5 et l'extension inférieure 4. Le capteur ultrasonore 6 est ainsi plaqué en permanence contre la mâchoire et suit les mouvements de celle-ci.

Le casque 1 comporte des moyens d'analyse (en l'occurrence un processeur exécutant un logiciel spécialisé) pour analyser le signal émis par le capteur ultrasonore, pour en déduire une parole de l'utilisateur (même en articulation silencieuse).

Selon l'invention, le casque 1 est équipé de moyens pour connaître la position angulaire relative du bras par rapport à l'arceau du casque. En l'occurrence, ces moyens comportent un capteur de rotation 8 disposé au niveau de l'articulation du bras 5, dont le signal est transmis aux moyens d'analyse. Grâce à ce capteur, il est possible de connaître à tout moment l'angle du bras par rapport au reste du casque 1, et donc d'en déduire la position angulaire du capteur ultrasonore par rapport au casque. En supposant que le casque est immobile par rapport au crâne de l'utilisateur, il est donc possible d'en déduire l'angle d'incidence du rayonnement ultrasonore par rapport au fond de la cavité buccale. Cette information est alors mise à profit par les moyens d'analyse pour mieux exploiter le signal généré par le capteur ultrasonore.

Selon maintenant un deuxième mode particulier de réalisation illustré à la figure 2, le dispositif de l'invention comporte ici une sonde ultrasonore 20 comportant une série de transducteurs ultrasonores synchronisés et qui peut être tenue à la main, être maintenue en position par un collier, une jugulaire, ou encore être disposée en extrémité d'un téléphone portable.

La sonde ultrasonore 20 est destinée à être utilisée de concert avec un objet porté par l'utilisateur en étant immobile par rapport au crâne de l'utilisateur, en l'occurrence ici une paire de lunettes 22 portée par l'utilisateur de la sonde ultrasonore 20.

La sonde ultrasonore 20 et la paire de lunettes 22 sont équipées de moyens de localisation (respectivement référencés 21 et 23) permettant de déterminer une position de la sonde ultrasonore 20 relativement à la paire de lunettes 22. Celle-ci étant supposée immobile vis-à-vis du crâne de l'utilisateur, on en déduit une position de la sonde ultrasonore par rapport au crâne.

Selon un mode particulier de réalisation, les moyens de localisation comportent des accéléromètres à trois voies 21,23 portés respectivement par la sonde ultrasonore 20 et la paire de lunettes 22.

Les accéléromètres sont de façon connue en soi utilisés comme inclinomètres pour déterminer deux angles d'inclinaison d'un trièdre de référence par rapport à un axe vertical défini par la direction locale de la pesanteur. Ainsi, les accéléromètres permettent de déterminer, à une rotation près autour de l'axe vertical précité, les positions angulaires de la sonde ultrasonore 20 et de la paire de lunettes 22 respectivement trièdre R1 d'axes x1, y1, z1 pour la sonde ultrasonore, et trièdre R2 d'axes x2, y2, z2 pour la paire de lunettes).

A titre d'illustration, sur la figure 3, on a représenté comment, à partir des trois composantes de l'accélération ax, ay, az mesurées qui, en l'absence d'un mouvement accéléré significatif, vérifient la relation $a_x^2 + a_y^2 + a_z^2 = g^2$, on peut reconstituer deux angles permettant de repérer angulairement le trièdre par rapport à la verticale définie par la gravité (à une rotation près autour de cette verticale). Les angles θ et φ vérifient les relations :

$$\begin{cases} a_x = \|g\| \sin\varphi \cos\theta \\ a_y = \|g\| \sin\varphi \sin\theta \\ a_z = \|g\| \cos\varphi \end{cases}$$

Pour lever l'incertitude liée à la position angulaire autour de l'axe vertical, on peut compléter les accéléromètres avec des gyromètres fournissant l'angle manquant. A défaut, il convient, avant utilisation, de caler les trièdres R1 et R2 entre eux de façon à pouvoir repérer après calage les positions de la sonde ultrasonore 20 et de la paire de lunettes 22, et d'en déduire par différence leur position relative.

De préférence, les accéléromètres 21, 23 sont placés sur la sonde ultrasonore 20 et la paire de lunettes 22 de sorte qu'un plan de l'un des trièdres soit sensiblement coplanaire avec un plan de l'autre trièdre lorsque l'utilisateur tient la sonde ultrasonore dans une position de référence (comme indiqué sur la figure 2 où les plans (x1, z1) et (x2, z2) sont coplanaires). Une procédure de calage préalable à l'utilisation de la sonde ultrasonore 20 peut être prévue pour guider l'utilisateur (par exemple par émission de bips sonores) afin qu'il place la sonde ultrasonore dans la position de référence, de sorte à caler angulairement la sonde ultrasonore 20 par rapport à la paire de lunettes 22 avant toute utilisation du dispositif.

Par la suite, tout mouvement relatif de la sonde ultrasonore 20 par rapport à la paire de lunettes 22 sera détecté par une simple comparaison des mesures des accéléromètres 21,23 ce qui permet à tout instant de connaître la position relative de la sonde ultrasonore 20 par rapport au crâne, et donc l'orientation de la sonde ultrasonore par rapport au fond de la cavité buccale. Les accéléromètres ainsi disposés forment ensemble des moyens de localisation de la sonde par rapport au crâne de l'utilisateur.

Pour ce faire, des moyens de communication (filaires ou à distance) reliés aux accéléromètres permettent de faire remonter les positions mesurées vers des moyens de calcul associés aux moyens d'analyse pour, en temps réel, déterminer l'orientation angulaire de la sonde ultrasonore et en tenir compte pour l'analyse du signal de la sonde. Les moyens de calcul sont par exemple constitués d'un processeur implanté dans le téléphone portable au bout duquel la sonde ultrasonore est positionnée.

L'invention n'est pas limitée à ce qui vient d'être décrit, mais englobe toute variante entrant dans le cadre défini par les revendications.

En particulier, bien que dans les modes de réalisations décrits, le ou les transducteurs ultrasonores (capteurs, sonde) sont utilisés pour sonder la cavité buccale et ainsi suivre les mouvements de la langue, on pourra plus généralement utiliser le ou les transducteurs ultrasonores pour sonder l'appareil phonatoire, par exemple le mouvement des lèvres.

Le dispositif de l'invention pourra comporter d'autres capteurs générant des signaux pouvant aider à la reconnaissance ou à la reconstruction de la parole, par exemple une caméra filmant le mouvement des lèvres.

Bien sûr, d'autres moyens de localisation peuvent être utilisés dans le cadre de l'invention, comme par exemple des centrales inertielles associées respectivement à l'objet fixe par rapport au crâne et à la sonde ultrasonore. En outre, on pourra bien évidemment utiliser comme élément fixe tout objet qui, en utilisation reste immobile par rapport au crâne, comme par exemple un casque, une oreillette, un bonnet ...

## Revendications

1.  Dispositif portable de reconnaissance et/ou de reconstruction de la parole par sondage ultrasonore de l'appareil phonatoire, le dispositif comportant au moins un transducteur ultrasonore (6,20) pour générer une onde ultrasonore et recevoir une onde réfléchie par l'appareil phonatoire de l'utilisateur, des moyens d'analyse d'un signal généré par le transducteur ultrasonore, et des moyens de localisation (8 ; 21, 23) pour déterminer une position relative du transducteur ultrasonore par rapport au crâne de l'utilisateur et **caractérisé en ce que** les moyens de localisation comportent un capteur de position angulaire d'un bras (5) qui est articulé sur un casque (1) et au bout duquel le transducteur ultrasonore est porté, le capteur de position angulaire étant disposé au niveau de l'articulation du bras.

2.  Dispositif portable de reconnaissance et/ou de reconstruction de la parole par sondage ultrasonore de l'appareil phonatoire, le dispositif comportant au moins un transducteur ultrasonore (6,20) pour générer une onde ultrasonore et recevoir une onde réfléchie par l'appareil phonatoire de l'utilisateur, et des moyens d'analyse d'un signal généré par le transducteur ultrasonore, et des moyens de localisation (8 ; 21, 23) pour déterminer une position relative du transducteur ultrasonore par rapport au crâne de l'utilisateur et **caractérisé en ce que** les moyens de localisation comportent un premier moyen de localisation (21) solidaire du transducteur ultrasonore, un deuxième moyen de localisation (23) solidaire d'un objet porté par l'utilisateur pour être fixe par rapport au crâne de celui-ci, et des moyens de calcul pour en déduire une position relative de la sonde par rapport au crâne, les moyens de localisation comportant chacun au moins un accéléromètre à trois voies.

## Patentansprüche

1.  Tragbare Vorrichtung zur Erkennung und/oder Rekonstruktion der Sprache durch Ultraschallabtastung des Sprechapparats, wobei die Vorrichtung mindestens einen Ultraschallwandler (6, 20) umfasst, um eine Ultraschallwelle zu erzeugen und eine von dem Sprechapparat des Benutzers reflektierte Welle zu empfangen, Analysemittel zum Analysieren eines von dem Ultraschallwandler erzeugten Signals, sowie Lokalisierungsmittel (8; 21, 23) zum Bestimmen einer relativen Position des Ultraschallwandlers relativ zum Schädel des Benutzers, und **dadurch gekennzeichnet, dass** die Lokalisierungsmittel einen Winkelpositionssensor zum Erfassen der Winkelposition eines Arms (5) umfassen, der an einem Helm (1) angelenkt ist und an dessen Ende der Ultraschallwandler getragen wird, wobei der Winkelpositionssensor im Bereich der Gelenkverbindung des Arms angeordnet ist.

2.  Tragbare Vorrichtung zur Erkennung und/oder Rekonstruktion der Sprache durch Ultraschallabtastung des Sprechapparats, wobei die Vorrichtung mindestens einen Ultraschallwandler (6, 20) umfasst, um eine Ultraschallwelle zu erzeugen und eine von dem Sprechapparat des Benutzers reflektiert Welle zu empfangen, Analysemittel zum Analysieren eines von dem Ultraschallwandler erzeugten Signals, sowie Lokalisierungsmittel (8; 21, 23) zum Bestimmen einer relativen Position des Ultraschallwandlers relativ zu dem Schädel des Benutzers und **dadurch gekennzeichnet, dass** die Lokalisierungsmittel ein erstes Lokalisierungsmittel (21) umfassen, das mit dem Ultraschallwandler verbunden ist, ein zweites Lokalisierungsmittel (23), das mit einem Objekt verbunden ist, das von dem Nutzer getragen wird, um relativ zu dessen Schädel ortsfest zu sein, sowie Rechenmittel, um daraus eine relative Position der Sonde in Bezug auf den Schädel abzuleiten, wobei die Lokalisierungsmittel jeweils mindestens einen Drei-Wege-Beschleunigungsmesser umfassen.

## Claims

1. A portable device for recognizing and/or reconstructing speech by ultrasound probing of the vocal apparatus, the device including at least one ultrasound transducer (6, 20) for generating an ultrasound wave and for receiving a wave reflected by the user's vocal apparatus, analysis means for analyzing a signal generated by the ultrasound transducer, and locating means (8; 21, 23) for determining the position of the ultrasound transducer relative to the skull of the user, the device being **characterized in that** the locating means comprise an angular position sensor for sensing the angular position of an arm (5) hinged to a headset (1) and having the ultrasound transducer carried at the end thereof, the angular position sensor being arranged at the hinge of the arm.

2. A portable device for recognizing and/or reconstructing speech by ultrasound probing of the vocal apparatus, the device including at least one ultrasound transducer (6, 20) for generating an ultrasound wave and for receiving a wave reflected by the user's vocal apparatus, analysis means for analyzing a signal generated by the ultrasound transducer, and locating means (8; 21, 23) for determining the position of the ultrasound transducer relative to the skull of the user, the device being **characterized in that** the locating means comprise first locating means (21) secured to the ultrasound transducer, second locating means (23) secured to an item worn by the user so as to be stationary relative to the user's skull, and calculation means for deducing therefrom the position of the probe relative to the skull, each of the locating means comprising at least one three-channel accelerometer.

Fig.1

Fig.2

Fig.3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **BRUCE DENBY ; MAUREEN STONE.** SPEECH SYNTHESIS FROM REAL TIME ULTRASOUND IMAGES OF THE TONGUE. *IEEE International Conference on Acoustics, Speech, and Signal Processing -ICASSP04,* 17 Mai 2004
- **STONE, M. ; DAVIS, E.** A Head and Transducer Support System for Making Ultrasound Images of Tongue/Jaw Movement. *Journal of the Acoustical Society of America,* 1995, vol. 98 (6), 3107-3112

- **SCOBBIE, J. M. et al.** Head-Probe Stabilisation in Ultrasound Tongue Imaging Using a Headset to Permit Natural Head Movement. *Proceedings of the Eight International Seminar on Speech Production (ISSP),* 10 Décembre 2008